# EUROPEAN PATENT SPECIFICATION

(11) **EP 2 042 124 B1**
(45) Date of publication and mention of the grant of the patent: **03.11.2010**
(21) Application number: 08102414.3
(22) Date of filing: 07.03.2008
(51) Int. Cl.: A61F 2/16

(54) **Intraocular lens**
Intraokulare Linse
Lentille intraoculaire

(30) Priority: 27.09.2007 US 862244
(43) Date of publication of application: 01.04.2009
(73) Proprietor: Alcon Research, Ltd., Fort Worth, TX 76134-2099 (US)
(72) Inventor: Morgan, Drew, Fort Worth, TX 76109 (US)
(74) Representative: Hanna, Peter William Derek

(56) References cited:
- WO-A-2004/107020
- US-A1- 2005 125 055
- US-A1- 2005 187 621
- US-A1- 2006 142 855

## Description

This invention relates to intraocular lenses (IOLs) and more particularly to single piece IOLs.

### Background of the Invention

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens.

When trauma, age or disease cause the lens to become less transparent, vision deteriorates because of the diminished light which can be transmitted to the retina. This deficiency in the lens of the eye is medically known as a cataract. The treatment for this condition is surgical removal of the lens and implantation of an artificial lens or IOL.

While early IOLs were made from hard plastic, such as polymethylmethacrylate (PMMA), soft, foldable IOLs made from silicone, soft acrylics and hydrogels have become increasingly popular because of the ability to fold or roll these soft lenses and insert them through a smaller incision. Several methods of rolling or folding the lenses are used. One popular method is an injector cartridge that folds the lenses and provides a relatively small diameter lumen through which the lens may be pushed into the eye, usually by a soft tip plunger. The most commonly used injector cartridge design is illustrated in U.S. Patent No. 4,681,102 (Bartell), and includes a split, longitudinally hinged cartridge. Similar designs are illustrated in U.S. Patent Nos. 5,494,484 and 5,499,987 (Feingold) and 5,616,148 and 5,620,450 (Eagles, et al.). In an attempt to avoid the claims of U.S. Patent No. 4,681,102, several solid cartridges have been investigated, see for example U.S. Patent No. 5,275,604 (Rheinish, et al.), 5,653,715 (Reich, et al.), and U.S. Patent No. 5,947,976 (Van Noy, et al).

These prior art devices were intended to inject an IOL into the posterior chamber of an aphakic eye through a relatively large (approximately 3.0 mm or larger) incision. Surgical techniques and IOLs have been developed that allow the entire surgical procedure to be performed through much smaller incisions, 2.4 mm and smaller. As a result, IOLs capable of being rolled or folded small enough to fit through such a small incision are desirable. To accomplish this goal, the IOL must be made thinner, or debulked, yet still have a refractive power of 30D or more. Thinning the center of the optic thus requires thinning the edge of the optic as well. Disadvantages of IOLs with such thin cross-sections, particularly when made from a soft, foldable material, are that the optic edge is very fragile and easily damaged during insertion, particularly when a plungered insertion device is used. In addition, the haptic/optic junction is very thin and weak and such lenses can become unstable in the eye. One solution to this stability problem is to implant a stabilizing ring in the capsular bag and locate the IOL within this ring. Such a construction is illustrated in US Patent Publication No. 2007/0010881 (Soye, et al.). While a two part lens system is effective in providing a very thin, stable IOL that can be implanted through a very small incision, the two components can be more difficult to implant that a single component lens.

US 2005/187621 A1 (Advanced Medical Optics, Inc.) describes an IOL having peripheral planar transitional region extending around the edge of the optic, to which a thicker rim is integrally disposed to provide an extra supporting structure.

US 2006/0142855 A1 (Bausch & Lomb Inc.) describes an IOL having a sharp-edged peripheral rim around the edge of the optic, but the optic edge is greater than 0.1 mm in thickness (between 0.1 and 0.3 mm) and the main peripheral supporting structure is provided by four thickened haptics (between 0.2 and 0.4 mm) disposed around the peripheral rim.

Accordingly, a need continues to exist for a single-piece, stable IOL that can be implanted through a very small incision.

### Brief Summary of the Invention

The present invention improves upon prior art by providing an IOL as disclosed in the appended claims having a depressed inner optic area and a thickened or raised peripheral outer lip or rim integrally formed with the optic, in accordance with claims which follow. Such a design reduces the mass of the IOL, making the lens easier to insert in a very small incision, without damaging the optic or compromising the stability of the IOL.

It is accordingly an objective of the present invention to provide a stable IOL.

It is a further objective of the present invention to provide a stable IOL that is more easily inserted through a very small incision in the eye.

It is yet a further objective of the present invention to provide an IOL having a depressed inner optic area and a thickened or raised peripheral outer lip or rim integrally formed with the optic.

Other objectives, features and advantages of the present invention will become apparent with reference to the drawings, and the following description of the drawings and claims.

### Brief Description of the Drawings

FIG. 1 is an enlarged top plan view of the IOL of the present invention.
   FIG. 2 is an enlarged cross-sectional view of an IOL not part of the present invention taken at line A-A in FIG. 1.
   FIG. 3 is an enlarged cross-sectional view of an embodiment of the IOL of the present invention taken at line A-A in FIG. 1.

### Detailed Description of the Preferred Embodiments

IOL 10 of the present invention generally includes optic 12 and at least two haptics 14. IOL 10 may have an overall length of any suitable dimension, with between 10.5 millimeters (mm) and 14.0 mm being preferred and 12.5 mm being most preferred. Optic 12 and haptics 14 are molded in a single piece from the same material. The material used to make IOL 10 may be any soft (not part of the invention) biocompatible material capable of being folded. Suitable materials are the hydrogel, silicone or soft acrylic (as disclosed in the appended claims) materials described in U.S. Pat. Nos. 5,411,553 (Gerace, et al.), 5,403,901 (Namdaran, et al.), 5,359,021 (Weinschenk, III, et al.), 5,236,970 (Christ, et al.), 5,141,507 (Parekh) and 4,834,750 (Gupta). Optic 12 has an anterior side 24 and a posterior side 26 and may be of any suitable diameter, with between 4.5 mm and 7.0 mm being preferred and between 5.5 mm to 6.0 mm being most preferred. Optic 12 may also be elliptical or oval. As shown in the following table, the maximum thickness of optic 12 will vary depending on the dioptic power desired and the index of refraction for the material used, but for with edge 18 being 0.1 mm thick and rim 30 or 30' being approximately 0.30 mm thick, the central thickness of optic 12 generally will be between 0.19 mm and 1.19 mm for a power range of between 6D and 71 D, but for the IOL as claimed the central thickness is 0.39 mm or less, with between 0.37 mm and 0.39 mm being most preferred.

| **Power (D)** | **Central Optic Thickness (mm)** |
|---|---|
| 71.00 | 1.19 |
| 61.25 | 1.02 |
| 39.25 | 0.68 |
| 24.25 | 0.45 |
| 21.75 | 0.41 |
| 20.75 | 0.40 |
| 19.75 | 0.39 |
| 19.00 | 0.37 |
| 6.25 | 0.19 |

The principal design criteria for IOL 10 is to minimize the thickness of optic 12 for any given diameter and power of optic 12 so as to minimizing the size of the surgical incision required to implant IOL 10. The material used to make optic 12 may be modified to absorb ultraviolet radiation, or any other desired radiation wavelength, such as blue or violet light.

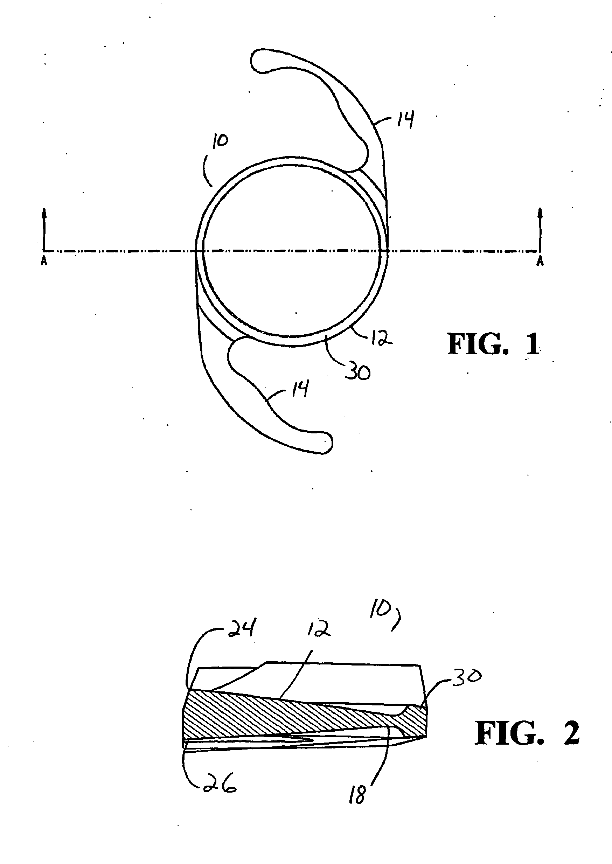

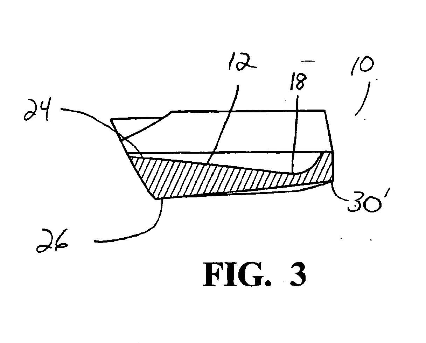

As best seen in FIGS. 2 and 3, optic 12 contains peripheral lip or rim 30 or 30', respectively, integrally formed as part of optic 12 and extending substantially or complete around peripheral edge 18 of optic 12. Rim 30 may be centered axially on optic 12 (not part of the invention), as seen in FIG. 2, or rim 30' is axially located anteriorly on optic 12 (as disclosed in the appended claims). Such a construction allows the reduction in thickness of optic 12 while maintaining the stability of IOL 10 in the eye.

While certain embodiments of the present invention have been described above,these descriptions are given for purposes of illustration and explanation. Variations, changes, modifications and departures from the systems and methods disclosed above may be adopted without departure from the scope of the present invention.

## Claims

1. An intraocular lens (10), comprising:
a) an optic (12) having a radial extent defined by an edge (18), the edge of the optic having a thickness of approximately 0.1 mm;
b) a peripheral rim (30,30') extending around the edge (18) of the optic disposed directly adjacent to the edge of the optic and integrally formed as part of the optic and located anteriorly on the optic (12); and
c) a plurality of haptics (14) extending from the rim and integrally formed with the optic and the rim; and wherein the intraocular lens (10) is made from a soft acrylic material,
**characterized in that**;
the optic (12) has a maximum thickness of 0.39 mm,

2. The intraocular lens of claim 1, wherein the peripheral rim (30,30') is thickened relative to the edge (18) of the optic (12).

3. The intraocular lens of claim 1, wherein the peripheral rim (30,30') is approximately 0.30 mm thick.

4. The intraocular lens of claim 1, wherein the optic (12) has a maximum thickness of between 0.19 mm and 0.39 mm.

## Patentansprüche

1. Intraokularlinse (10), umfassend:
a) eine optische Linse (12) mit einer von einer Kante (18) definierten radialen Ausdehnung, wobei die Kante der optischen Linse eine Dicke von ungefähr 0,1mm aufweist;
b) einen Umfangsrand (30, 30'), der sich um die Kante (18) der optischen Linse erstreckt und direkt neben der Kante der optischen Linse angeordnet und als Teil der optischen Linse einstückig mit ihr gebildet ist und sich vorderhalb an der optischen Linse (12) befindet, und
c) eine Vielzahl von Haptiken (14), die sich vom Umfangsrand erstrecken und einstückig mit der optischen Linse und dem Umfangsrand gebildet sind; und wobei die Intraokularlinse (10) aus einem weichen Acrylmaterial besteht.

2. Intraokularlinse nach Anspruch 1, wobei die Dicke des Umfangrandes (30, 30') entsprechend der Kante (18) der optischen Linse (12) gestaltet wird.

3. Intraokularlinse nach Anspruch 1, wobei der Umfangsrand (30, 30') ungefähr 0,30mm dick ist.

4. Intraokularlinse nach Anspruch 1, wobei die optische Linse (12) eine maximale Dicke von zwischen 0,19mm und 0,39mm aufweist.

## Revendications

1. Lentille intraoculaire (10), comprenant :
a) une optique (12) ayant une portée radiale définie par un bord (18), le bord de l'optique ayant une épaisseur d'approximativement 0,1 mm ;
b) un rebord périphérique (30, 30') s'étendant autour du bord (18) de l'optique disposé directement adjacent au bord de l'optique et formé solidairement comme une partie de l'optique et situé de façon antérieure sur l'optique (12) ; et
c) une pluralité d'haptiques (14) s'étendant depuis le rebord et solidairement formés avec l'optique et le rebord ; et dans laquelle la lentille intraoculaire (10) est constituée d'un matériau acrylique mou ;
**caractérisée en ce que** :
l'optique (12) a une épaisseur maximale de 0,39 mm.

2. Lentille intraoculaire selon la revendication 1, dans laquelle le rebord périphérique (30, 30') est épaissi par rapport au bord (18) de l'optique (12).

3. Lentille intraoculaire selon la revendication 1, dans laquelle le rebord périphérique (30, 30') est épais d'approximativement 0,30 mm.

4. Lentille intraoculaire selon la revendication 1, dans laquelle l'optique (12) a une épaisseur maximale comprise entre 0,19 mm et 0,39 mm.
